# EUROPEAN PATENT APPLICATION

(11) **EP 3 141 180 A1**
(43) Date of publication of application: **15.03.2017**
(21) Application number: 15184644.1
(22) Date of filing: 10.09.2015
(51) Int. Cl.: A61B 5/00

(54) **DIAGNOSTIC DEVICE FOR DETERMINING ELASTIC PROPERTIES OF SOFT TISSUE**

(71) Applicant: ETH Zurich, 8092 Zurich (CH)
(72) Inventor: MAZZA, Edoardo, 5430 Wettingen (CH); BADIR, Sabrina, 8057 Zürich (CH)

(57) **Abstract**

A device for measuring the elastic deformability of soft tissue (5) comprises a probe (1) with a port and a probe channel (13), the port being configured to be attached to a pressure unit that provides a vacuum and the probe channel (13) being arranged in a housing (16). The housing (16) extends beyond the probe (1) along a distal direction and has a lower closed loop surface (17) so as to form a cavity (19). The probe channel (13) is in connection with the pressure unit and extends preferably partly into the cavity (19) along the distal direction. A plug (110, 127) is displaceably arranged within the cavity (19). The plug (110, 127) is displaceable along a proximal direction towards a distal end (14) of the probe channel (13) when the housing (16) is attached to the soft tissue (5). The plug (110, 127) completely covers the distal end (14) of the probe channel (13) when the housing (16) is attached to the soft tissue (5) in a final position.

## Description

### TECHNICAL FIELD

The present invention relates to a device for determining elastic and / or visco-elastic properties of soft tissue, in particular to a device for measuring the deformability of the uterine cervix for the prediction of preterm birth.

### PRIOR ART

The determination of mechanical properties of soft tissue, especially soft tissue, is important in several areas such as in cosmetics, pharmacy and dermatology. For example, the determination of the elasticity of soft tissue provides information on physiological or medical conditions, as a deformed healthy or hydrated soft tissue will quickly move back into its undeformed state, whereas dehydrated soft tissue will return into its undeformed state only slowly. Various ways for assessing soft tissue type and soft tissue condition are known.

From WO 2014/029509 A1, a device for determining elastic properties of soft tissue or scalp is known that comprises a probe having a probe pin and a measurement system for registering a displacement of the probe. The probe pin is provided in a probe chamber having an opening that allows for contact of the probe pin with the scalp or the soft tissue and is biased to be flush with the opening or biased to protrude from the opening. The pin measures the displacement of the soft tissue under forces induced by an under pressure in the chamber.

EP 2 301 436 A1 discloses a device for measming the elastic deformability of soft tissue, where a measuring probe is provided that exerts pressure on the soft tissue surface on a probe channel. A light barrier is disposed in the region of the probe channel and the change in light intensity serves as a measure for the deformation of the soft tissue surface.

The determination of the elasticity of soft tissue, particularly of human soft tissue such as the female uterine cervix showed to be important also in the field of preterm birth. Preterm birth is a major cause of perinatal morbidity and mortality. Moreover, preterm babies are at increased risk for newborn health complications as well as lasting disabilities. In pregnancy, the uterine cervix serves two major functions. First, it retains its physical integrity by remaining firm during pregnancy as the uterus dramatically enlarges. This physical integrity is critical so that the developing fetus can remain in the uterus until the appropriate time for delivery. Second, in preparation for labor and delivery, the cervix softens and becomes more distensible, a process called cervical ripening. These changes are required for cervical dilation, labor and delivery of a fetus. Thus, the uterine cervix has to provide mechanical resistance to ensure a normal development of the fetus, where cervical softening should occur progressively throughout pregnancy. The assessment of an early cervical ripening thus enables a treatment option for preterm labor or other applications where better knowledge of the soft tissue conditions of the cervix is paramount.

Aspiration methods and devices for assessment of viscoelastic properties of soft tissues which are based on detecting an inflection point on a pressure-time plot when air is aspirated from a cavity placed over the tissue sample are known from US 7,955,278 B1. Thereby, a small diameter tube through which air aspiration is conducted is ultimately closed off by tissue being drawn into the cavity causing an abrupt change in pressure slope.

A procedure for the mechanical characterization of the uterine cervix during pregnancy is disclosed in Badir, S. et al, Journal of the Mechanical Behaviour of Biomedical Materials, 27, 143-153 (2013). There, a tube is connected to a pump used to generate a progressive vacuum, where one end of the tube is placed on the cervix. With progressive vacuum increase, cervical tissue is sucked into the tube until it closes a pipe used to evacuate the tube. The pressure required to deform the tissue for a specified distance is proportional to the stiffness of the cervix. A digital camera and glass fibers are integrated in the tube in order to facilitate the placement of the tube on the cervix.

The direct contact between the tissue and the aspirating tube bears a high risk of injury. In order to obtain reproducible results, these measuring procedures have to be performed under strictly controlled conditions. The camera-guided placement of the tube requires an experienced gynecologist, since he has to look at a monitor while simultaneously placing the probe on the cervix with the help of the monitor. Additionally, devices that need high-tech components such as a camera are expensive and must be maintained regularly.

### SUMMARY OF THE INVENTION

It is an object of the present invention to provide a device for measuring the elastic deformability of soft tissue that enables a simple and reliable measurement and where the risk of injury is minimized.

This object is accomplished by the device for measuring the elastic deformability of soft tissue according to claim 1. The device comprises a probe with a port and a probe channel. The port is configured to be attached to a pressure unit that provides a vacuum and the probe channel is arranged in a housing. The housing extends beyond the probe along a distal direction and has a lower closed loop surface so as to form a cavity. The probe channel is in connection with the pressure unit and has a distal end. Distal end can optionally extend at least partly into the cavity along the distal direction. It is also a solution that the probe channel ends flush with the probe body.

A plug is displaceably arranged within the cavity. The plug is displaceable along a proximal direction towards a distal end of the probe channel when the housing is attached to the soft tissue. The plug completely covers the distal end of the probe channel when the housing is attached to the soft tissue in a final position.

In that the plug completely covers the distal end of the probe channel after a complete attachment of the housing to the soft tissue, a direct contact between the soft tissue and the probe channel is prevented. That is to say, the plug can serve the purpose of a closure for the probe channel. This has the advantage that not the soft tissue is closing the typically peaked and sharp distal end of the probe channel, but the plug. Thereby, any injuries of the soft tissue that could occur by the probe channel are avoided.

In this context, a direction from the housing toward the cavity, which is substantially parallel to a longitudinal center axis of the probe, is defined as the distal direction. The opposing direction is referred to as the proximal direction.

In an initial position, optionally provided by an abutment or a spring connection, the plug preferably protrudes at least partly from the housing along the distal direction. Thus, before attachment of the housing to the soft tissue, the plug can distally protrude out of the housing and can be brought into contact with the soft tissue before the housing contacts the soft tissue. Hence, the housing can be brought into contact with the soft tissue after the plug contacts the soft tissue. This position is referred to as the starting position, i.e. the position where the housing is just brought into contact with the soft tissue and the plug starts displacing into the cavity.

For example, the spring connection can be established by a spring-loaded element as will be explained further below and which holds the plug in the housing. It is also possible that the plug is prevented from falling out of the housing in the initial position by an abutment such as a recess or a lug formed in the housing, which can interact with a corresponding recess or lug formed in the plug.

The cavity formed by the housing can thus be understood as an interior space which is proximally delimited by the distal end of the probe, which is laterally surrounded by a side wall of the housing and which is distally opened so that the plug can at least partly protrude out of the housing in the initial position.

In that the plug initially protrudes from the housing, it can serve as a guide means for a facilitated, controlled and exact placement of the housing on the soft tissue. Such a proper placement also enables controlled, exact and therefore reproducible measurements of the deformability of the soft tissue without the need of any further technical means such as e.g. a camera. Furthermore, elaborate controls or calibrations and unnecessary high contact forces are avoided.

Through the contact of the plug with the soft tissue, the plug can be displaced along the proximal direction. That is to say, when the housing is attached to the soft tissue, the initially protruding plug can first be brought into contact with the soft tissue and can displace thereupon along the proximal direction.

Preferably, an under pressure is applied to the probe channel upon displacement of the plug along the proximal direction, the housing thereby being increasingly attached to the soft tissue.

As already mentioned, the probe channel is in connection with the pressure unit and preferably such, that a displacement of the plug along the proximal direction into the cavity initiates the aspiration of air through the probe channel. Thus, once the plug is placed on the soft tissue and starts displacing into the cavity, an under pressure is generated in the cavity which progressively adheres the housing to the surface of the soft tissue until the housing is completely attached to the soft tissue.

Preferably, the soft tissue is increasingly deformable into the cavity along the proximal direction when the housing is completely attached to the soft tissue, wherein the plug is displaced along the proximal direction by the deforming soft tissue. Thus, once the housing is completely attached to the soft tissue, a tight contact between the soft tissue and the housing is established and a continuously increasing under pressure, i.e., a vacuum, can be generated in the cavity. Said under pressure may suck the surface of the soft tissue into the cavity. Thereby, the soft tissue sucked into the cavity is deformed such, that it increasingly pushes the plug further into the cavity of the housing and towards the probe channel.

It is preferred that the under pressure is interrupted when the plug completely covers the distal end of the probe channel. Hence, once the soft tissue is deformed to such an extent that it has pushed the plug to the distal end of the probe channel, the plug is in a final position and preferably closes the probe channel and thereby interrupts a further aspiration of air out of the cavity.

The dimensions of the cavity and of the plug are preferably chosen such, that the plug is fully received within the cavity in the final position.

Preferably, the distance along which the soft tissue has to deform into the cavity so as to push the plug from the initial position all the way up to the probe channel corresponds to a specified distance. Thereby, the final position can be defined and the pressure required to deform the soft tissue over the specified distance can be used to determine the elasticity of the soft tissue. In particular, the pressure required to deform the soft tissue over the specified distance is proportional to the stiffness of the cervix.

The probe can comprise a further probe channel which preferably has a distal end which does not extend into the cavity along the distal direction and which is not in connection with the pressure unit. The probe channel and the further probe channel can each be connected to a pressure sensor. The deformability of the soft tissue can be measured as a pressure difference between the pressure sensors of the probe channel and of the further probe channel.

Thus, the pressure required to deform the soft tissue over the specified distance can be determined via the difference between the under pressure in the probe channel connected to the pressure unit and the further probe channel which is not connected to the pressure unit, which pressure is representative for the stiffness of the cervix. It is preferred that the plug covers both the distal end of the probe channel and that of the further probe channel in the final position, but only closes the probe channel.

Preferably, at least one spring-loaded element is arranged in the cavity, wherein the spring-loaded element connects the proximal end of the plug with a distal end of the probe in such a manner, that the plug is displaceable under the load of the spring along the proximal direction. Hence, the spring-loaded element can be used for biasing the plug in the housing. For example, the spring-loaded element can be a spring link that pushes the plug at least partly out of the housing in the initial position, and which spring link is increasingly pushed together once the plug has contacted the soft tissue and increasingly displaces towards to probe channel.

The distal end of the plug being arranged on the side facing the soft tissue preferably has a convex surface. It is preferred, that the housing is essentially of a cylindrical shape and defines a housing diameter, and that the plug is essentially of a cylindrical shape and defines a plug diameter, wherein the housing diameter is larger than the plug diameter.

As a result, the cavity can have a clear with that corresponds to the diameter of the housing and a clear length that corresponds to the distance between the distal end of the probe and the distal end of the housing and the cylindrical plug can be arranged spaced away from an inside wall of the cylindrical housing.

The distal end of the cylindrical plug preferably has a diameter which is larger than that of the proximal end. In particular, the diameter of the distal end of the cylindrical plug is such, that it is in close proximity to the inside wall of the cylindrical housing upon displacement of the plug into the cavity of the housing.

However, it is also possible that the plug has an essentially half-spherical shape, wherein the distal end corresponds to the spherical side having a convex surface that faces the soft tissue. The proximal end of the plug can have a radial thickening which is arranged in immediate proximity to an inner wall of the housing. Preferably, the spring-loaded element, in particular a spring link, is then attached to the radial thickening of the plug. More preferably, two spring-loaded elements, in particular spring links, are attached to two opposing sides of the radially thickened proximal end of the plug with respect to the longitudinal center axis of the probe.

It is preferred that the proximal end of the plug has an elongation, wherein the elongation extends along the proximal direction and is adapted to at least partially surround the probe channel and/or the further probe channel. Said elongation preferably is in the form of a hollow cylinder and has a diameter that is smaller than the diameter of the distal end of the cylinder. Preferably, a proximal end of said hollow cylinder is open so as to receive the probe channel and the further probe channel upon displacement of the plug towards the probe. The spring-loaded element, in particular a spring link, can be attached to the proximal end of the elongation. More preferably, two spring-loaded elements, in particular spring links, are attached to two opposing sides of the proximal end of the elongation with respect to the longitudinal center axis of the probe. When the plug is in the final position, the probe channel and / or the further probe channel are preferably completely received within the hollow cylinder of the elongation.

It is preferred that the housing is essentially in the form of a hollow cylinder and comprises at least one recess in the area of its distal end, the at least one recess being in connection to the pressure unit. In order to connect the at least one recess with the pressure unit, the housing can comprise at least one port that extends from the recess at the distal end of the housing to the proximal end of the housing, where it is adapted for being connected to the pressure unit. It is preferred that the conducts leading to the pressure unit are separated enabling a better control of the under pressure in the probe channel, while maintaining an essentially constant under pressure in the hollow cylinder leading to the recess(es) connecting the surrounding soft tissues.

The recess can have the form of a small circular hole, where it is preferable that at least two small holes, more preferably a number of small holes being spaced at an equal distance from each other, are formed in the distal end of the housing. It is also possible that the recess is in the form of a circular recess that is surrounding the distal end of the housing along a circumferential direction. Since the recess(es) can be in connection with the pressure unit, a negative pressure can be applied to the soft tissue via said recess(es). While the soft tissue is deformed into the cavity, the surrounding soft tissue is fixed at the recess(es) so as to avoid any lateral shift of the surrounding soft tissue towards the cavity. That is to say, the recess(es) serve the purpose of a vacuum clamplugg of the housing on the soft tissue and thus reduce any measurement uncertainty related to unknown friction coefficient.

In the region of the distal end of the housing, the surface of the inner wall of the housing can be slanted towards the inside of the cavity along the proximal direction, where the recess(es) are preferably formed on said slanted surface of the inner wall. Preferably, the inner wall surface is slanted towards the inside of the cavity at an angle between 25 to 65 degrees, more preferably between 35 to 55 degrees, most preferably about 45 degrees, with respect to a plane being perpendicular to the longitudinal center axis of the probe. The recess(es) can be formed in that slanted surface such, that they extent only partly over the slanted surface or such, that they extent essentially over the whole slanted surface of the housing. It is also possible that the recess(es) are formed into the slanted surface which merges into a rounded distal end of the housing, where the recess(es) are directed towards the inside of the cavity and where only the rounded distal end of the housing, but not the recess(es) are contacting the soft tissue. The rounded distal end of the housing enables a smooth deformation of the soft tissue into the cavity.

The device preferably further comprises a measuring unit adapted for measuring a remaining distance between the initial position, where the plug is first contacting the soft tissue, and the final position, where the plug completely covers the distal end of the probe channel.

The measuring unit can serve the purpose of providing a continuous feedback on the remaining distance between the plug and the probe channel and thus supports an easy and reliable attachment of the probe on the soft tissue.

The measuring unit can comprise at least one first electrode being in connection with the plug and at least one second electrode being in connection with the probe channel, wherein the first electrode and the second electrode increasingly overlap with increasing displacement of the plug along the proximal direction so as to generate an increased capacity. That is to say, the remaining distance between the plug and the probe channel can be measured by means of a capacity measurement between the two electrodes.

The measuring unit can comprise at least one sliding contact being in connection with the plug and a resistance covering the probe channel, wherein the resistivity between the sliding contact and the resistance decreases with increasing displacement of the plug along the proximal direction. That is to say, the remaining distance between the plug and the probe channel can be measured by means of a resistivity measurement between the sliding contact and the resistance.

The measuring unit can comprise at least one coil being in connection with the plug, preferably a plunger coil being in connection with the proximal end of the plug, wherein the probe channel comprises a magnetic material, and wherein the inductivity between the coil and the magnetic material increases with increasing displacement of the plug along the proximal direction. That is to say, the remaining distance between the plug and the probe channel can be measured by means of an inductivity measurement between the coil and the magnetic material.

The measuring unit can comprise a light source and a light detector that are arranged within the cavity, wherein the light source sends light to the plug and the light detector detects the light reflected from the plug, and wherein the amount of reflected light increases with increasing displacement of the plug along the proximal direction. That is to say, the remaining distance between the plug and the probe channel can be measured by means of an optical measurement using the light source and the light detector.

The measuring unit can comprise several membranes that are attached proximally to the plug, wherein said membranes are punctured before der plug completely covers the distal end of the probe channel, the pressure in the device thereby being neutralized to atmospheric pressure. That is to say, the remaining distance between the plug and the probe channel can be measured by means of a mechanical measurement where the membranes are progressively punctured while the plug is progressively displaced into the cavity. Preferably, the membranes are arranged within the elongation of the hollow cylindrical plug and are punctured by the probe channel and / or the further probe channel when the plug is pushed towards these channels. Depending on the degree of displacement, a particular membrane closes the probe channel and a negative pressure is created within the cavity, or the particular membrane is punctured by the probe channel and the negative pressure is neutralized to atmospheric pressure.

The measuring unit can comprise a plurality of sealing rings, wherein the probe channel comprises a plurality of probe channel perforations and the plug comprises one plug perforation. A negative pressure can be generated within the probe channel if one of the sealing rings overlies with the plug perforation, and the negative pressure can be neutralized if the plug perforation overlies with the probe channel perforation. The cycle of negative pressure and neutralized pressure is indicative of the displacement of the plug along the proximal direction. That is to say, remaining distance between the plug and the probe channel can be measured by means of a mechanical measurement where the sealing rings are successively covering plug perforation while the plug is progressively displaced into the cavity.

The measuring unit can comprise an additional housing that is arranged displaceably around the housing. Preferably, the additional housing protrudes over the housing along the distal direction, and the housing protrudes over the additional housing along the proximal direction. A decreasing protrusion of the housing relative to the additional housing can be indicative of the displacement of the additional housing along the proximal direction. That is to say, the remaining distance between the plug and the probe channel can be measured by means of a mechanical measurement where the remaining distance between the plug and the probe can be determined according to the remaining distance between the additional housing and the housing.

The device preferably further comprises a control unit that can be separable from the probe. It can be implemented in the handle. It is preferred that the pressure unit and / or the pressure sensors are arranged within or attached to the control unit. It is preferred that the probe is made of plastic, or the front part as disposable part is of plastic and can be attached to a permanent part of the device, comprising pressure unit and control unit and being e.g. with metallic parts and a connector element, with which the disposable front part can be connected, especially with a screw or bayonet connection.

It is possible to arrange a filter, in particular an air filter, between the probe and the control unit such, that the probe can be removed completely from the device. A probe made of plastic has the advantage that it is cheap and easy to manufacture. That is to say, the probe can serve the function of a disposable probe.

Furthermore, in that a disposable probe is provided which can be separated from the control unit, which comprises rather expensive components such as a pressure unit or pressure sensors, costs and time that are otherwise needed for detailed hygiene precautions such as sterilization can be minimized or even avoided.

The probe channel with the distal end, optionally extending at least partly into the cavity along the distal direction will be an additional disposable part together with an air filter, to avoid contact of fluids with the probe housing and the pump.

As described above, the measuring results sensed by the pressure sensors can be an under pressure value that is related to a certain degree of deformation of the soft tissue. Additionally, it is possible to correlated said under pressure value to a clinically relevant parameter such as the risk of a preterm birth, i.e., said risk being negative or positive, or high, moderate or low, respectively. It is possible that the measuring result and / or a clinically relevant parameter are displayed on the probe or on a display of the control unit.

A method for measuring the elastic deformability of soft tissue by applying an under pressure on the soft tissue with a probe as described above comprises the steps of: i) Placing the probe in the initial position on the soft tissue, wherein only the protruding plug contacts the soft tissue, and wherein the plug starts to displace along the proximal direction; ii) Applying the under pressure once the plug has started to displace along the proximal direction; iii) Displacing the housing along the distal direction until the distal end of the housing contacts the soft tissue, wherein the plug continuously displaces along the proximal direction towards the distal end of the probe channel; and iv) Interrupting the under pressure once the plug completely covers the distal end of the probe channel in the final position, wherein the deformability of the soft tissue is measured as a pressure difference between the initial position and the final position

It is to be noted that the device described herein is not limited to the prediction of preterm birth only, but can have many different applications. For example, it can be used for the observance and quantification of the healing process of soft tissue after soft tissue transplantation. Another example would be its application in the development of drugs, where mechanical properties of soft tissue are to be manipulated.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a perspective view of a device comprising a probe, a pressure unit and a control unit for measuring the elastic deformability of soft tissue according to the present invention;
- Fig. 2: shows a perspective view of the probe according to Fig. 1 comprising a housing and a displaceable plug;
- Fig. 3: shows a front view of the probe and the housing according to Fig. 2;
- Fig. 4: shows a series of sketches illustrating the attachment of the probe to the soft tissue with a displaceable plug;
- Fig. 5: shows a series of sketches illustrating the attachment of the probe to the soft tissue with a displaceable plug according to a further embodiment;
- Fig. 6: shows a schematic illustration of a housing attached to the soft tissue in a starting position;
- Fig. 7: shows a schematic illustration of the housing according to Fig. 6 in a final position;
- Fig. 8: shows a schematic sketch illustrating the attachment of the probe according to Fig. 4, further comprising a measuring unit;
- Fig. 9: shows a schematic sketch illustrating the attachment of the probe according to Fig. 4, further comprising a measuring unit according to a further embodiment;
- Fig. 10: shows a schematic sketch illustrating the attachment of the probe according to Fig. 4, further comprising a measuring unit according to a further embodiment;
- Fig. 11: shows a schematic sketch illustrating the attachment of the probe according to Fig. 4, further comprising a measuring unit according to a further embodiment;
- Fig. 12: shows an enlarged view of the region A of Fig. 11;
- Fig. 13: shows a schematic sketch illustrating the attachment of the probe according to Fig. 4, further comprising a measuring unit according to a further embodiment;
- Fig. 14: shows a schematic sketch illustrating the attachment of the probe according to Fig. 4, further comprising a measuring unit according to a further embodiment;
- Fig. 15: shows a schematic sketch illustrating the attachment of the probe according to Fig. 4, further comprising a measuring unit according to a further embodiment;
- Fig. 16: shows a schematic sketch illustrating an embodiment of an apparatus comprising the probe according to Fig. 4; and
- Fig. 17: shows a schematic sketch illustrating a further embodiment of an apparatus comprising the probe according to Fig. 4.

### DESCRIPTION OF PREFERRED EMBODIMENTS

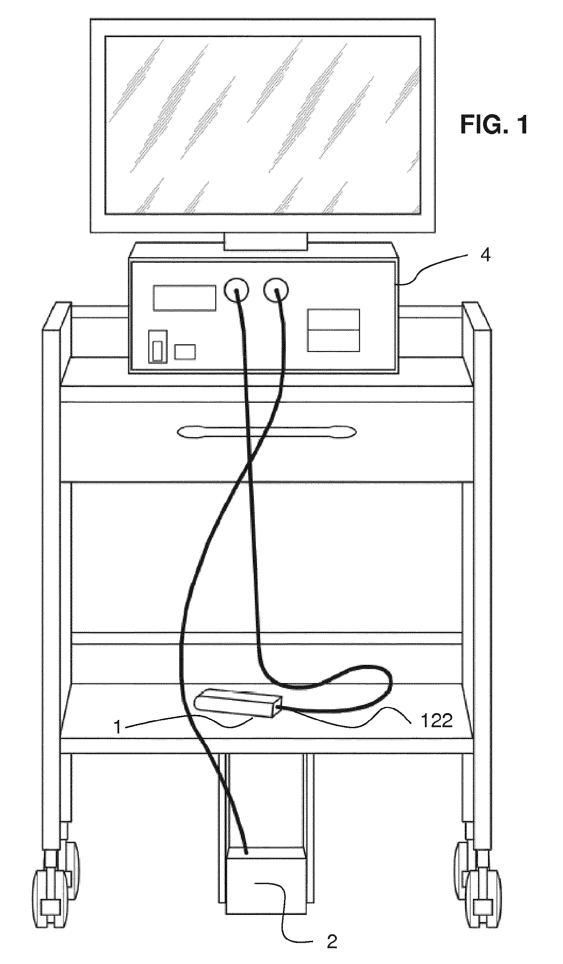

A device for measuring the elastic deformability of soft tissue according to the present invention is shown in Fig. 1. The device comprises a probe 1 which is adapted to be connected to a pressure unit 2 and a control unit 4, individual aspects of which are discussed in the following.

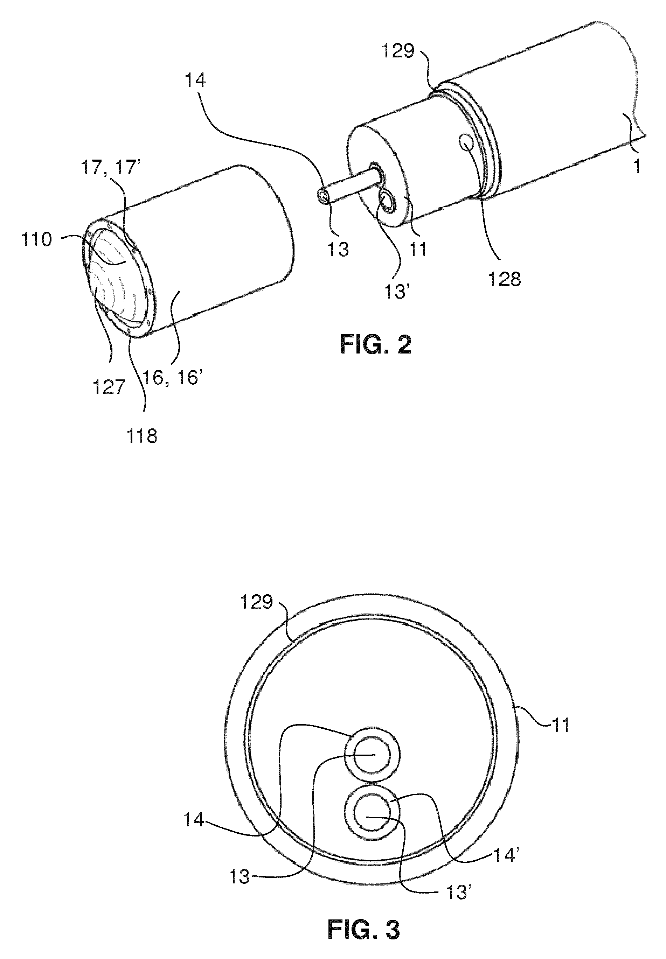

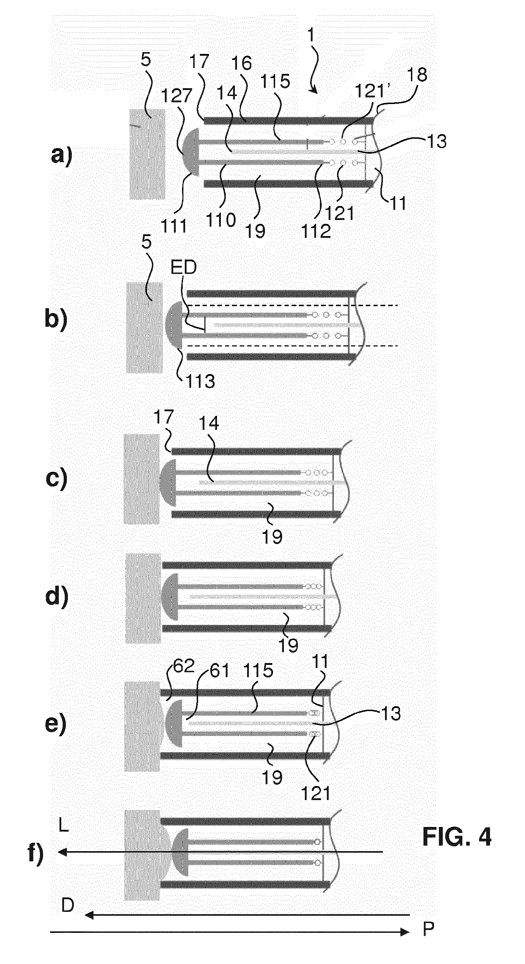

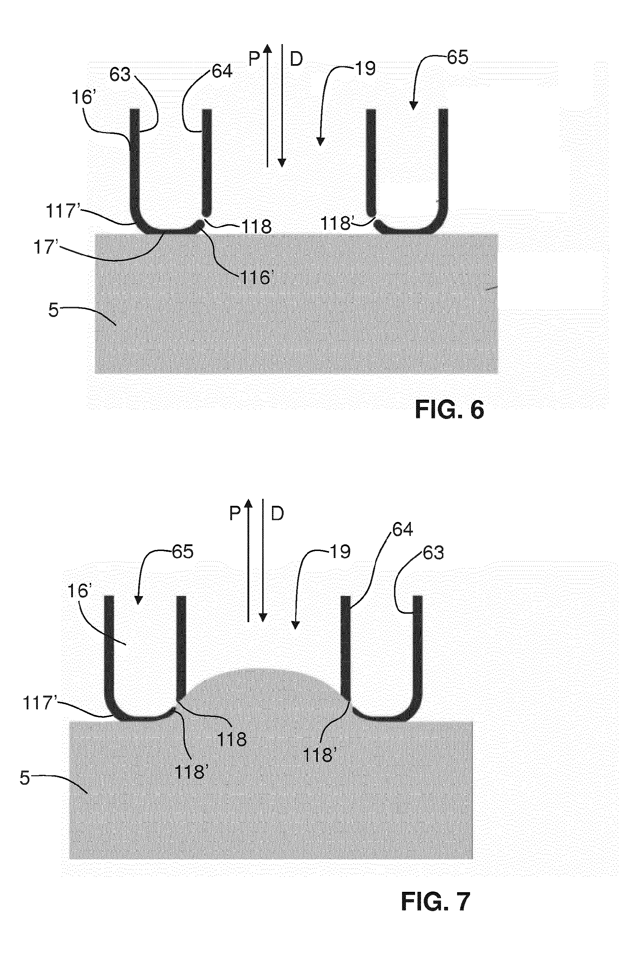

The probe 1, which is to be arranged in a housing 16, 16', as well as the housing 16, 16' are each shown individually in Fig. 2. The housing 16, 16' is in the form of a hollow cylinder and is adapted to receive the cylindrical probe 1. The housing 16, 16' extends beyond the probe 1 along a distal direction D (see inter alia Figs. 4 or 6) and has a lower closed loop surface 17, 17' so as to form a cavity 19 between said lower end surface and the cylindrical probe body. The probe 1 comprises a probe channel 13, hereafter referred to as suction channel, and a further probe channel 13', hereinafter referred to as measuring channel, respectively. Both the suction channel 13 and the measuring channel 13' extend substantially parallel to one another through the probe 1 and have distal ends 14, 14' which each extend partly into the cavity 19 along the distal direction D. Moreover, each channel 13, 13' is connected to a pressure sensor (not shown), whereas only the suction channel 13 is in connection with the pressure unit 2. In particular, the probe 1 has a port at its proximal end 12 by means of which the probe 1 can be connected to the pressure unit 2.

The lower end 17, 17' of the housing 16, 16' comprises eight recesses 118 or holes in the lower surface. The hollow cylinder, see Fig. 6 and 7 provides a suction channel to the proximal end of the housing 16, 16'. There, an internal opening is provided in the inner wall facing the longitudinal axis of the cylinder and covers, when the housing 16, 16' is mounted on the probe 1, the further suction channel 128. The further suction channel 128 is connected with the pressure unit, preferably with a different tubing.

Housing 16, 16' can be connected with the probe 1 through a pair of threads 129, so that the further suction channel 128 is provided near the internal opening of housing 16 to provide an unrestricted fluid path. Plug 110 is shown extending over the front distal end 17 with its convex tip head 127 as will be explained in connection with Fig. 4pp.

A front view of the probe 1 without a housing in the form of a cylinder 16 and in the form of a hollow cylinder 16', respectively, are displayed in Fig. 3. In the region of the distal end 17, 17' of the housing 16, 16', a plug or pin 110, 110' is displaceably arranged within the cavity 19. In an initial position, before attachment of the housing 16, 16' to the soft tissue 5, the pin 110, 110' protrudes partly from the housing 16, 16' along the distal direction D. The term plug and pin are used synonymously in the present description for element 110. In fact the plug can be any element which can be displaced along the longitudinal axis of the instrument and has a at least partially essentially planar or or slightly convex rounded outer surface in direction of the soft tissue and has at least a partially planar surface adapted to cover and finally close the suction channel in an airtight way. It is preferred that the suction channel 13 is provided centrally in order to be closed by the middle portion of the plug 110, but this is not prerequisite. Further suction channel 13' with the encompassing tubing 14' is provided near the suction channel 13, but can be rek

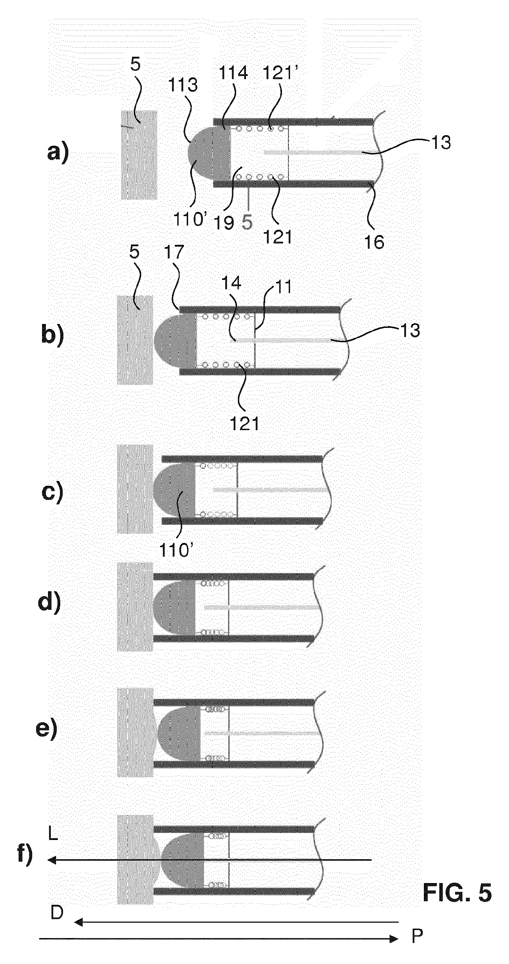

Figs. 4 and 5 show a series of sketches illustrating the attachment of the probe 1 to the soft tissue with a displaceable pin 110, 110'. These sketches show the housing 16 in the form of a hollow cylinder with lower opening or recesses 118 in the front distal surface.

Therefore, when the probe 1 is placed on the soft tissue 5, only the distal end 111, 111' of the protruding pin 110, 110' contacts the soft tissue 5 at first. Thereafter, the housing 16, 16' is displaced along the distal direction D until also the distal end 17, 17' of the housing 16, 16' contacts the soft tissue 5, or, in other words, the pin 110, 110' is displaced along the proximal direction P into the cavity 19 until also the distal end 17, 17' of the housing 16, 16' contacts the soft tissue 5, respectively. Thereby, as soon as the pin 110, 110' starts displacing along the proximal direction P, the aspiration of air through the suction channel 13 is initiated by means of the port 122 of the probe 1, which is in connection with the pressure unit 2. It is also possible to wait until pin 110, 110' touches the soft tissue 5 through measurement of the position of the pin 110, 110' as will be explained later.

When both, the distal end 111, 111' of the pin 110, 110' as well as the distal end 17, 17' of the housing 16, 16' is contacting the soft tissue 5, a starting position is defined. The starting position can be detected, if the pressure unit 2 is already providing under pressure in cavity 19 by the control unit through detecting a change of the pressure measured with probe 14. This change can be a sudden change towards lower pressure since no further air is guided or sucked into the partial cavities 62 and 61, i.e. the external cavity portion 62 between the housing 16 and the bottom and side surface of pin 110, 110', and the internal cavity portion 61 between the tip of probe channel 14 and the distal part of pin 110, 110'. The continuous application of under pressure to the suction channel 13 then generates a vacuum in the cavity 19 which progressively adheres the housing 16, 16' to the surface of the soft tissue. At the same time, it also continuously displaces the pin 110, 110' further into the cavity 19, since the air is initially sucked from the area around the open tip 14 of the suction channel 13 and there is a pressure gradient between that zone 61 and the volume between the distal end 111, 111' of the pin 110, 110' and the soft tissue in zone 62. During said continuous displacement of the pin 110, 110', the portion of surface of the soft tissue 5 under the closed loop of the distal end housing 17, 17' is continuously displaced into the cavity 19, too. As soon as the pin 110, 110' reaches its final possible position, where the proximal end 112, 112' of the pin 110, 110' covers the distal end 14 of the suction channel 13, the aspiration of air through the suction channel 13 is interrupted, which is detected by the control unit.

Thus, the measurement of the deformability comprises a first stage, where a smooth placing of the probe 1 on the soft tissue 5 in the initial position takes place, a second stage, where an under pressure is continuously applied to the probe 1 in a controlled manner, and a third stage, where the suction through under pressure is interrupted by means of the pin 110, 110' reaching the final position. The deformability of the soft tissue 5 is then determined by means of calculating the pressure difference between the measured pressure at the time of the initial position and the measured pressure at the time of the final position. Thereby, a controlling or monitoring of these stages is performed by the control unit, which is adapted to sense the initial and / or starting and / or final position and which communicates with the pressure unit so as to initiate or interrupt the application of under pressure by means of the pressure unit depending on the status of the stages.

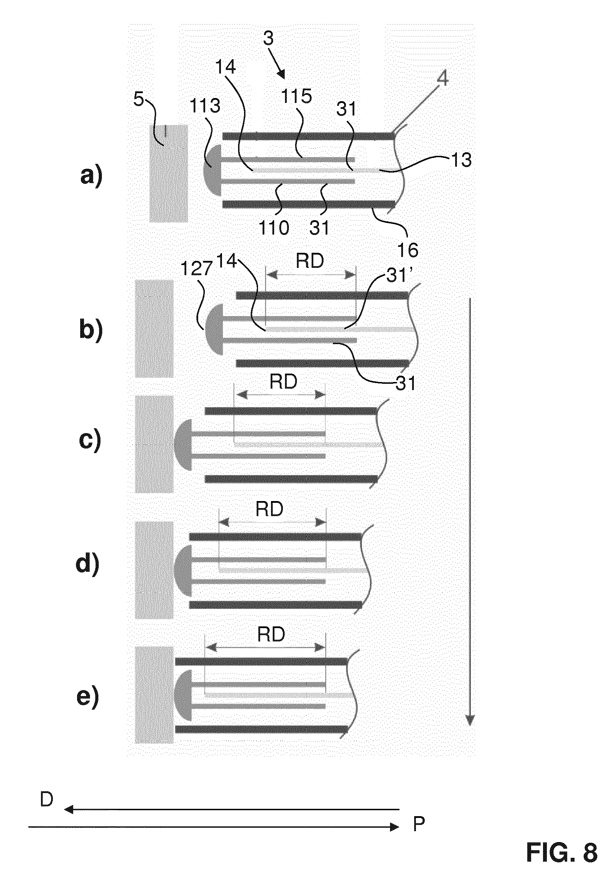

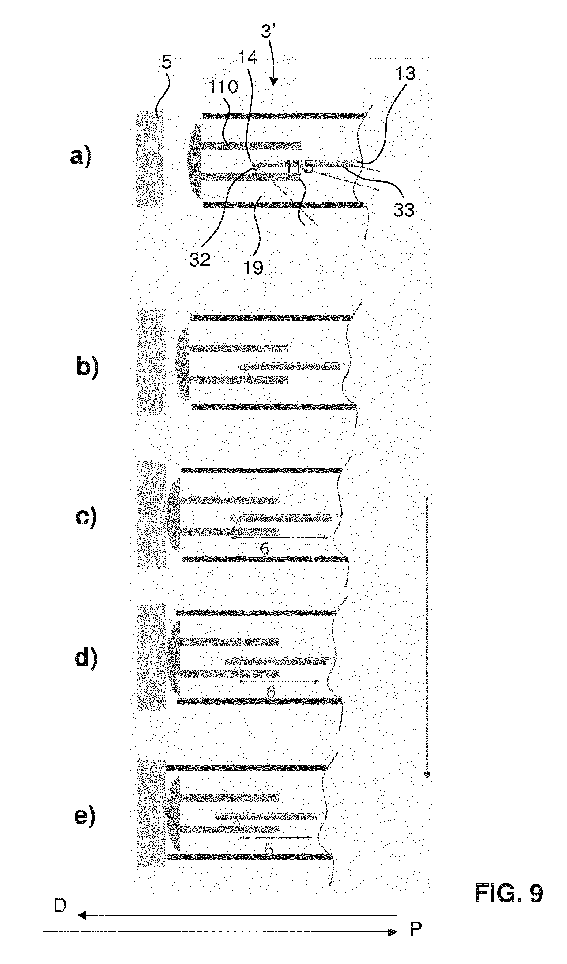

In particular, the attachment comprises preferably one continuous movement with a parallel or subsequent continuous onset of the pumping action. This movement is shown in the embodiments of Fig. 3 and Fig. 4 in several steps of specific points in time. Fig. 3 a) and Fig. 4a) show the probe device in a undetermined distance of the soft tissue 5 before its application; b) relates to the positioning of the probe 1 with the protruding pin 110, 110' close to the surface of the soft tissue 5; c) placement of the pin 110, 110' on the surface of the soft tissue 5, where a contact between the soft tissue 5 and the pin 110, 110' is sensed by means of the pin 110, 110' starting to displace along the proximal direction P into the cavity 19, thereby a negative pressure being applied to the suction channel 13 (or in other words an ongoing reduction of the distance between the front end surface 17 of the housing 16 and the soft tissue 5 surface); d) displacing the housing 16 along the distal direction D until the distal end of the housing contacts the soft tissue 5, or, in other words, displacing the pin 110, 110' along the proximal direction P into the cavity 19 until also the distal end 17 of the housing 16 contacts the soft tissue 5. Thereby, a vacuum being generated in the cavity 19 and a gentle positioning of the probe 1 is enabled until tight contact between the housing and the soft tissue is reached; e) continuously displacement of the pin 110, 110' into the cavity 19 through application of under pressure through the probe channel 13 while registering the remaining distance directly or indirectly between the displacing pin 110, 110' and the suction channel 13 (see also Figs. 8-15; RD in Fig. 8 grows whereas RD in Fig. 9 is reduced during movement of pin 110. 110'), the soft tissue 5 thereby being continuously deformed into the cavity 19 and the pin 110, 110' being continuously pushed towards the suction channel 13 by the deforming soft tissue since the lower pressure in partial cavity 61 creates a gradient to the external partial cavity 62; and finally f) maximum deformation of the soft tissue 5 into the cavity 19, the pin 110, 110' being thereby touching and closing of the suction channel 13.

In both Figs. 4 and 5, the housing 16 and the pin 110, 110' are essentially of a cylindrical shape, wherein the housing 16 has a housing diameter HD that is larger than a pin diameter PD, and wherein the distal end 111, 111' of the pin 110, 110' being arranged on the side facing the soft tissue 5 has a convex surface 113, 113'. It comprises an initial touch surface 127.

The proximal end 112 of the pin shown in Fig. 4 has an elongation 115 which extends along the proximal direction P. Said elongation 115 is in the form of a hollow cylinder and has a diameter ED that is smaller than the diameter PD of the distal end 111 of the pin 110. A proximal end of said hollow cylinder 115 is open so as to receive the suction channel 13 and the measuring channel 13' upon displacement of the pin 110 towards the probe 1. When the pin 110 is in a final position, the suction channel 13 and the measuring channel 13' are completely received within the hollow cylinder of the elongation 115 and the distal ends 14, 14' of which are completely covered by the proximal end 112 of the pin 110.

The pin 110' shown in Fig. 5 has an essentially half-spherical shape, wherein the distal end 111' corresponds to the spherical side having a convex surface 113' that faces the soft tissue 5, and wherein the proximal end 112' has a radial thickening 114 which is arranged in immediate proximity to the inner wall 116, 116' of the housing 16, 16'.

In both cases, two spring links 121, 121'are attached to the proximal end 112, 112' of the pin 110, 110' on two opposing sides with respect to a longitudinal center axis L of the probe 1. In Fig. 4, the spring links 121, 121' are attached to the proximal end of the elongation 115. In Fig. 5, the spring links 121, 121' are attached to the proximal end of the radial thickening 114 of the pin 110'. On their proximal side, the spring links 121, 121' are attached on two opposing sides of the distal end 11 of the probe 1 such, that the suction channel 13 and the measuring channel 13' are located midway between them. Thus, the spring links 121, 121' connect the proximal ends 112, 112' of the pin 110, 110' with the distal end 11 of the probe 1 in such a manner, that the pin 110, 110' is displaceable under the load of the spring along the proximal direction P. In an initial position, before attachment of the housing 16, 16' to the soft tissue 5, the spring links 121, 121' push the pin 110, 110' partly out of the housing 16, 16'. Upon contact is made between the distal end 111, 111' of the pin 110, 110' and the soft tissue 5, the pin 110, 110' progressively moves into the cavity 19 along the proximal direction P and the spring links 121, 121' are increasingly pushed together until the final position is reached, where the proximal end 112, 112' of the pin 110, 110' completely covers the suction channel 13.

Thereby, once the housing 16, 16' is contacting the soft tissue 5, a starting position is reached, where a tight contact between the soft tissue 5 and the housing 16, 16' is established and a continuously increasing under pressure is generated in the cavity 19. Said under pressure progressively sucks the surface of the soft tissue 5 into the cavity 19. The soft tissue 5 that is sucked into the cavity 19 is deformed such, that it touches the pin 110, 110' at the central part of contact surface 127 and increasingly pushes the pin 110, 110' further into the cavity 19 of the housing 16, 16' and towards the probe channel 13. Once the soft tissue 5 is deformed to such an extent that it has pushed the pin 110, 110' to the distal end 11 of the probe 1, the pin 110, 110' is in the final position and closes the suction channel 13 and thereby interrupts a further aspiration of air out of the cavity 19. In the final position, the pin 110, 110' is fully received within the cavity 19.

Moreover, the distance along which the soft tissue 5 has to deform into the cavity 19 so as to push the pin 110, 110' from the initial position all the way up to the suction channel 13 corresponds to a specified distance. Such a specified distance between contact of the soft tissue with the surrounding housing bottom can be 4 millimetres, especially between 2 and 6 millimeters, preferably between 3 and 5 millimeters, whereas the entire possible travel distance of the pin can be defined as being 20 millimetres, preferably between 10 and 25 millimeters. The pressure required to deform the soft tissue 5 over the specified distance is then used to determine the elasticity of the soft tissue 5, since the pressure required to deform the soft tissue 5 over the specified distance is proportional to the stiffness of the soft tissue 5. Herein, the deformability of the soft tissue 5 is measured as the pressure difference between the pressure sensors of the suction channel 13 and of the measuring channel 13'.

A schematic illustration of a housing 16' attached to the soft tissue 5 in a starting position and a final position is shown in Fig. 6 and 7, respectively. Said housing 16' is essentially in the form of a hollow cylinder and comprises recesses 118 in the form of small holes in the area of its distal end 17'. The recesses 118 are in connection with the pressure unit 2 so that a negative pressure can be applied to the soft tissue 5 via said recesses 118. In these Figures, the housing 16' comprises rounded distal ends 17' having an inner wall surface 116' being slanted towards the inside of the cavity 19 along and an outer wall surface 117' being slanted away from the housing 16', respectively. The slanted inner wall surface 116' is here slanted by about 45 degrees with respect to the flat, i.e., not deformed surface of the soft tissue 5. The recesses 118 are formed on the slanted surface of the inner wall 116', i.e. they are facing towards the inside of the cavity 19. After the housing 16' is placed on the surface of the soft tissue 5, however before an under pressure is generated in the cavity 19, only the rounded distal ends 17' of the housing 16', but not the recesses 118 are contacting the soft tissue 5, as follows from Fig. 6. After the housing 16' is completely attached to the soft tissue 5 and a continuously increasing under pressure is generated, the soft tissue 5 is sucked into the cavity 19. Thereby, the soft tissue 5 is also laterally sucked and fixed to the recesses 118, as follows from Fig. 7. In doing so, the recesses 118 serve the purpose of a vacuum clamping of the housing 16' on the soft tissue 5 and the rounded distal ends 17' of the housing 16' enable a smooth deformation of the soft tissue 5 into the cavity 19. In other words, the recesses 118 allow for vacuum clamping in order to avoid sliding of the soft tissue 5 and to obtain well-defined boundary conditions and at the same time an inherently safe soft tissue deformation.

Housing 16' of Fig. 6 or 7 comprises an outer wall 63 and an inner wall 64 with an intermediate hollow wall cavity 65. It is preferred that a number of recesses 118 are provided on the inner wall side 64 of the hollow housing 16' e.g. for an exterior housing diameter of 12 millimeter and an inner hollow cavity 19 with a diameter of 8 millimeters, the walls 63 and 64 define a hollow wall strength of 2 millimeters. Then the wall strength is e.g. 0,5 millimeters each with a hollow ring section of 1 millimeter, building up the hollow wall strength of 2 millimeters. There can be 8 to 16 holes in a regular circumferential distance of (in one embodiment) about 4 millimeter one from another (2*pi*r/number of holes). Of course the number of holes can be chosen to be 4, 8, 12, 16 or 24 or intermediate values. The recesses 118, 118' preferably have a diameter of 1 millimeters. In both Fig. 6 and 7 the central pin or plus is not shown; it would be in position e) or f) of Fig. 3 or 4.

The housing 16' comprises at least one suction opening at its proximal end. Since it is provided on the inside of housing 16' it is not visible on page 2. The probe body 1 comprises at least one further suction channel 128 near the distal end, wherein housing 16' and probe body 1 comprises complementary attachment elements to provide a detachable connection. In the embodiment of Fig. 2 these attachment elements are complementary threads 129; this can also be provided as a bayonet coupling, wherein the suction opening(s) and the further suction channel(s) 128 are positioned in a way that when the housing 16' is attached at the probe body 1 the suction opening(s) and the further suction channel(s) 128 are providing a medium outlet.

As has been mentioned above, the device can further comprise a measuring unit 3, 3', 3", 3"', 3"", 3""', 3""" adapted for measuring the remaining distance RD between the pin 110 and the probe channel 13, 13'. In particular, the measuring unit provides detailed information on the remaining distance RD during the steps a)-e) of Figures 4 and 5.

The mechanism to sense the first soft tissue contact, where an under pressure is applied to the suction channel, and the corresponding remaining distance RD until the pin 110 closes the suction channel 13, where the under pressure in interrupted and the pressure difference is determined, is illustrated in Figures 8-15. These Figures schematically illustrate the attachment of the probe 1 to the soft tissue 5 by means of a measuring unit 3, 3', 3", 3"', 3"", 3""', 3""" performing a capacity measurement, a resistivity measurement, an inductivity measurement, an optical measurement, and a pneumatic / mechanical measurement, respectively. For simplicity, no spring-link 121, 121' is shown in these Figures.

In particular, Fig. 8 displays a measuring unit 3, where the pin 110 is representing a first electrode 31 and the suction channel 13 is representing a second electrode 31', respectively. Fig. 8 a) shows the general layout while Fig. 8 b to e) shows that the first electrode 31 and the second electrode 31' increasingly overlap with increasing displacement of the pin 110 along the proximal direction P and thereby generate an increased capacity. The remaining distance RD is registered by means of the progressively increasing capacity until a specified value is reached that corresponds to the final position, where the pin 110 closes the suction channel 13.

Fig. 9 displays the measuring unit 3' according to a further embodiment with a similar sequence as in Fig. 8 with representations a) and b) to e), where the pin 110 is connected to a sliding contact 32 and where the outer radial surface of the suction channel 13 is covered by a resistance 33. The resistivity between the sliding contact 32 and the resistance 33 decreases with increasing displacement of the pin 110 along the proximal direction P, which allows the determination of the remaining distance RD.

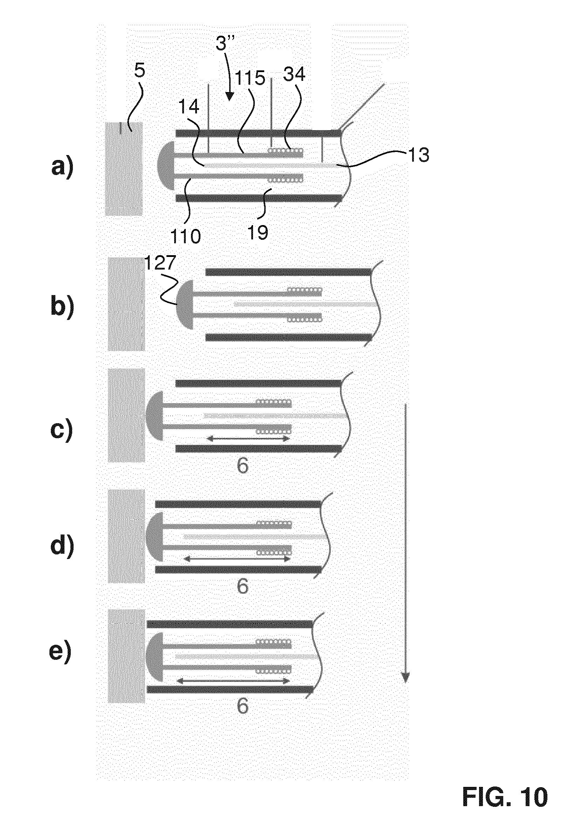

Fig. 10 displays the measuring unit 3" according to a further embodiment with a similar sequence as in Fig. 8 with representations a) and b) to e), where the proximal end 112 of the pin 110 is covered by a plunger coil 34 and where the suction channel 13 comprises a magnetic material. The inductivity between the coil 34 and the magnetic material of the suction channel 13 increases with increasing displacement of the pin 110 along the proximal direction P, and the remaining distance RD is registered by a progressively increasing inductivity to a specified value.

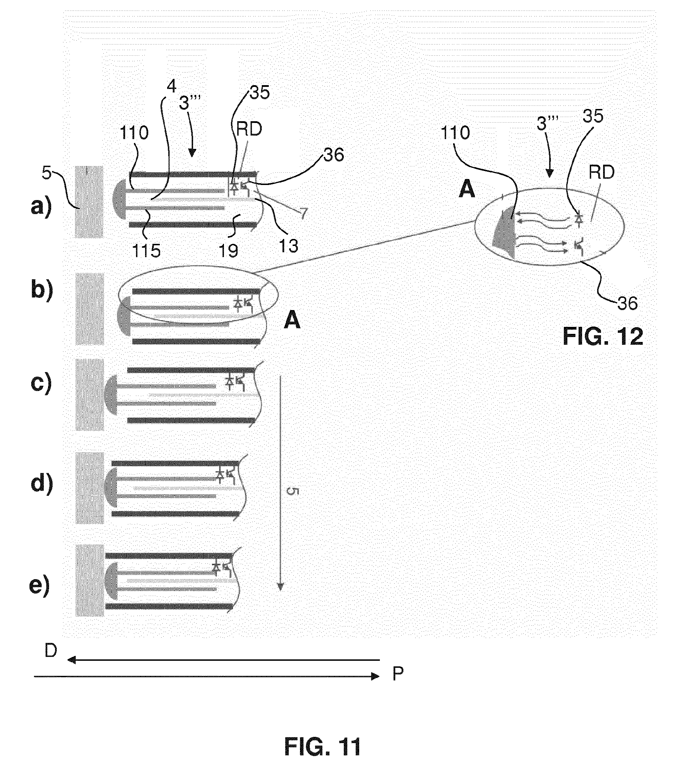

Fig. 11 displays the measuring unit 3"' according to a further embodiment, where the measuring unit comprises a light source 35 and a light detector 36 that are arranged within the cavity 19. The light source 35 sends light to the pin 110 and the light detector 36 detects the light reflected from the pin 110, and wherein the amount of reflected light increases with increasing displacement of the pin 110 along the proximal direction P. Thus, the remaining distance RD is registered by a progressively increasing light reflection to a specified value.

As can best seen in Fig. 12, which shows an enlarged view of the region A of Fig. 11, the light source 35 and the light detector 36 are both attached to the inside wall 116, 116' of the housing 16, 16' and extend laterally towards the longitudinal center axis L of the probe 1. Thereby, the light source 35 is placed further away than the light detector 36 along the distal direction D and is in immediate proximity to the proximal end 111 of the pin 110 once the pin is in its final position.

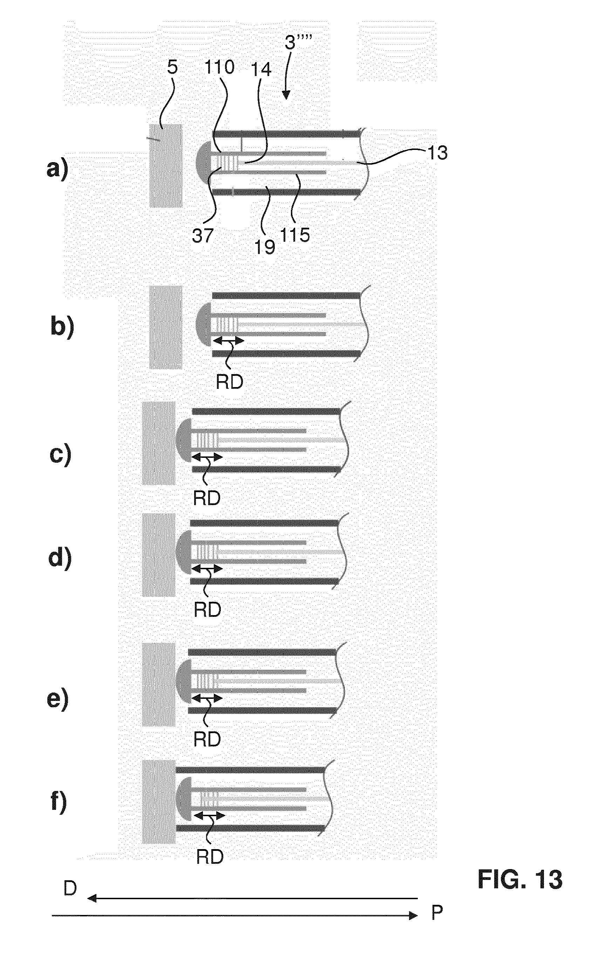

Fig. 13 displays the measuring unit 3"" according to a further embodiment, where the measuring unit comprises several membranes 37 that are arranged within the elongation 115 of the pin 110. Said membranes 37 are punctured before the pin 110 completely covers the distal end 14 of the suction channel 13, the pressure in the device thereby being neutralized to atmospheric pressure. Said membranes 37 are progressively punctured by the suction channel 13 when the pin 110 is pushed towards the suction channel 13. Depending on the degree of displacement, a particular membrane 37 closes the suction channel 13 and a negative pressure is created within the cavity 19, or the particular membrane 37 is punctured by the suction channel 13 and the negative pressure is neutralized to atmospheric pressure. After a few negative pressure / atmospheric pressure cycles, as shown in Fig. 13 f) the pin 110 is completely covering the distal end 14 of the suction channel 13.

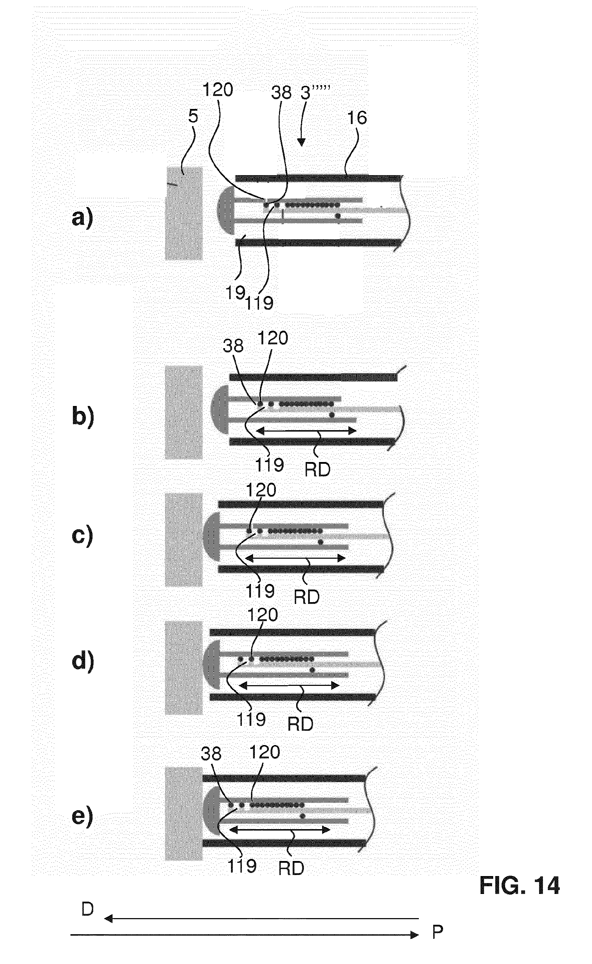

Fig. 14 displays the measuring unit according 3""' to a further embodiment, where the measuring unit comprises a plurality of sealing rings 38, the suction channel 13 comprises a plurality of suction channel perforations 119 and the pin 110 comprises one pin perforation 120, respectively. A negative pressure is generated within the suction channel 13 if one of the sealing rings 38 overlies with the pin perforation 120, and the negative pressure is neutralized if the pin perforation 120 overlies with the suction channel perforation 119, respectively. The cycle of negative pressure and neutralized pressure is indicative of the displacement of the pin 110 along the proximal direction P. After a few negative pressure / atmospheric pressure cycles, the pin 110 is completely covering the distal end 14 of the suction channel 13.

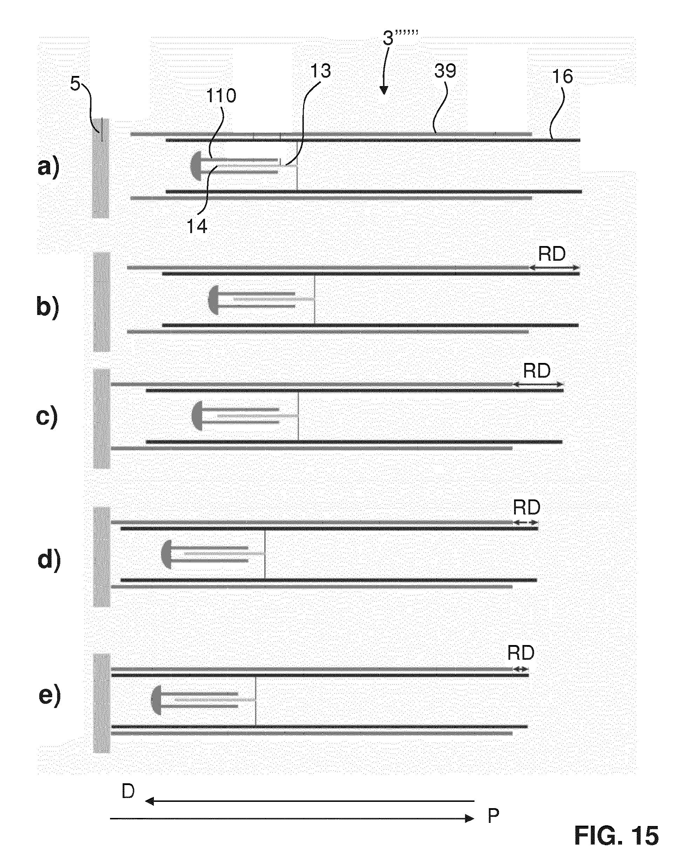

Fig. 15 displays the measuring unit 3""" according to a further embodiment, where the measuring unit comprises an additional cylindrical housing 39 that is surrounding the first cylindrical housing 16, 16' comprising the pin 110 and the probe 1. The additional housing 39 is arranged displaceably around the first housing 16, 16' and protrudes over the first housing 16, 16' along the distal direction D. The first housing 16, 16' protrudes over the additional housing 39 along the proximal direction P. When the probe 1 is going to be positioned on the soft tissue 5, the additional housing 39 is contacting the soft tissue 5 first. Upon contacting the soft tissue 5, the additional housing 39 progressively moves along the proximal direction P towards the proximal end 18, 18' of the first housing 16, 16'. Thereby, the protrusion of the first housing 16, 16' relative to the additional housing 39 is decreasing, i.e. the remaining distance RD is registered by the progressively decreasing relative displacement of the proximal end of the additional housing 39 with respect to a specified position such as the proximal end 18, 18' of the first housing 16, 16'.

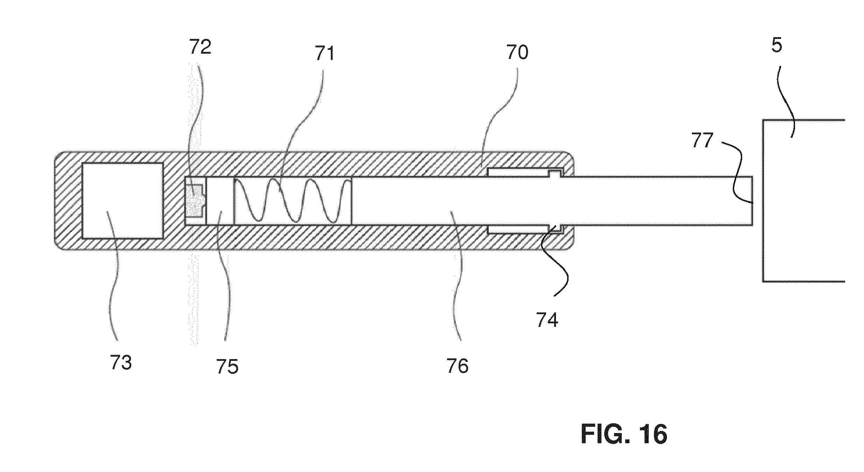

Fig. 16 shows a schematic sketch illustrating an embodiment of an apparatus with a probe handle 70 comprising the probe 1 according to Fig. 4, shown as moving probe tip 76. Handheld probe 1 is provided inside probe handle 70. The tip 76 can move along the axial direction of the probe, sliding inwards, into the handle 70, when the probe 1 is pushed with its front surface against the tissue 5. The moving probe tip 76 compresses a spring, which is anchored to a moving piston 75 that sits flush against a force sensor 72. The spring 71 dampens compressive forces, by the operator, against the tissue 5. The displacement at the force sensor 72 is transduced into an electrical signal quantifying the applied force.

The schematically shown front surface 77 comprises on one side the front distal end 17 of the hollow sleeve housing and on the other side the especially convex front surface 113 of the plug 110.

The probe 1 according to the embodiment has an integrated electronic 3D orientation sensor 73 working in the framework of the control unit. Information from this sensor 73 is used to determine and subsequently subtract the force component due to gravity that is also measured by the force sensor 72, whenever the probe 1 is not used horizontally.

The probe 1 is only shown as moving probe tip 76 with a front surface 77, since the teaching of the handheld probe handle 70 can be combined with various embodiments of the probe 1 as shown in Fig. 1 to 15. Moving probe tip 76 can comprise an abutment shoulder 74 provided within the probe handle 70 to retain the probe against the force of the spring 71.

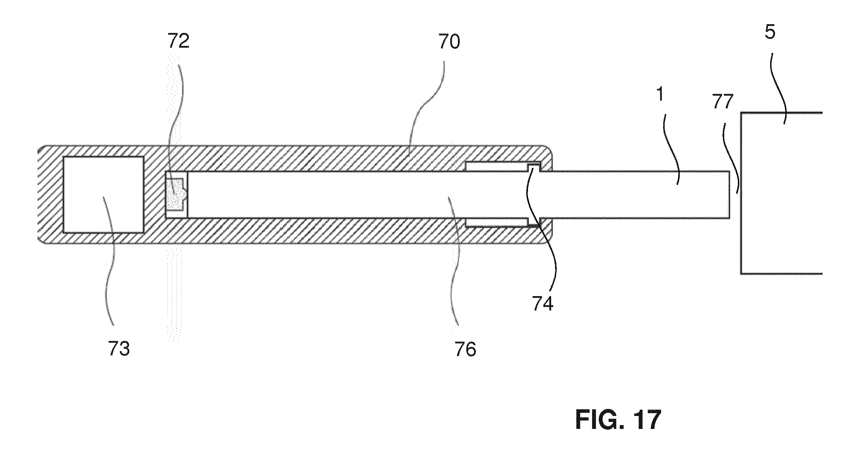

A further embodiment of a handheld probe handle 70 is shown in Fig. 17. The tip 70 can move along the axial direction of the probe 1, sliding inwards, into the handle, when the probe 1 is pushed against the tissue 5. The displacement at the force sensor 72 is transduced into an electrical signal quantifying the applied force as above. The probe 1 has a force sensor 73 directly an integrated electronic 3D orientation sensor. Information from this sensor 73 is also used to determine and subsequently subtract the force component due to gravity that is also measured by the force sensor 72, whenever the probe 1 is not used horizontally. Moving probe tip 76 can comprise an abutment shoulder 74 provided within the probe handle 70 to retain the probe and to have it guided in a free longitudinal movement against the force sensor 72.

It is also possible - and a different embodiment for Fig. 16 and Fig. 17 - that the element 76 of Fig. 16 and Fig. 17 comprises the elements sleeve 16, 16' from Fig. 8 to 15, suction channel 13 from Fig. 8 to 15 and an optional air filter as disposable parts connected with further parts within element 76 being connected with sleeve-like probe handle 70.

The idea of Fig. 16 is to have a "anchor base" (proximal part of 76) that moves, is connected to the spring 71, and is permanently in the handle 70. Then the disposable part (distal part of 76) is attached to that base. The base is also what connects the air tubing to the disposable part. In that solution, it will be an advantage to include a holding pin so that the user can attach the disposable part without always compressing the spring 71.

**LIST OF REFERENCE SIGNS**

| | | | |
|---|---|---|---|
| 1 | probe | 33 | resistance |
| 11 | distal end probe | 34 | coil |
| 12 | proximal end probe | 35 | light source |
| 13,13' | probe channel | 36 | detector |
| 14,14' | distal end probe channel | 37 | membrane |
| 15,15' | proximal end probe channel | 38 | sealing ring |
| 16,16' | housing | 39 | additional housing |
| 17,17' | distal end housing | 4 | control unit |
| 18,18' | proximal end housing | 5 | soft tissue |
| 19 | cavity | 61 | external partial cavity |
| 110,110' | pin | 62 | internal partial cavity |
| 111,111' | distal end pin | 63 | outer wall |
| 112,112' | proximal end pin | 64 | inner wall |
| 113,113' | convex surface | 65 | hollow wall cavity |
| 114 | radial thickening | 70 | probe handle |
| 115 | elongation | 71 | spring |
| 116,116' | inner wall housing | 72 | force sensor |
| 117,117' | outer wall housing | 73 | 3D orientation sensor |
| 118,118' | recess | 74 | abutment shoulder |
| 119 | probe channel perforation | 75 | moving piston |
| 120 | pin perforation | 76 | moving probe tip |
| 121,121' | spring-loaded element | 77 | front surface |
| 122 | port | | |
| 127 | contact surface of the plug | HD | housing diameter |
| 128 | further suction channel | PD | pin diameter |
| 2 | pressure unit | ED | elongation diameter |
| 3,3',3", 3"',3"", 3""',3"'"' | measuring unit | D | distal direction |
| | | P | proximal direction |
| | | L | longitudinal center axis |
| 31,31' | electrode | RD | remaining distance |
| 32 | sliding contact | | |

## Claims

1. Device for measuring the elastic deformability of soft tissue (5) comprising a probe (1) with a port (122), a probe channel (13), a pressure unit (2) and a control unit, the port (122) being configured to be attached to the pressure unit (2) that provides controlled by the control unit a vacuum and the probe channel (13) being arranged in a housing (16, 16'),
wherein the housing (16, 16') extends beyond the probe (1) along a distal direction (D) and has a lower closed loop surface (17, 17') so as to form a cavity (19), and
wherein the probe channel (13) is in connection with the pressure unit (2) and has a distal end (14), optionally extending at least partly into the cavity (19) along the distal direction (D),
**characterized in that** a plug (110, 110') is displaceably arranged within the cavity (19),
a pressure sensor is provided to determine the pressure in the cavity (19) and to communicate it to the control unit,
wherein the plug (110, 110') is displaceable along a proximal direction (P) towards the distal end (14) of the probe channel (13) and is configured to completely cover and close the distal end (14) of the probe channel (13), wherein the control unit is configured to determine the deformation of the soft tissue (5) using the pressure value when the plug (110, 110') covers and closes the distal end (14) defining a final position when the housing (16, 16') is attached to the soft tissue (5).

2. Device according to claim 1, **characterized in that** the housing (16') is essentially in the form of a double-walled (63, 64) hollow cylinder having a closed internal cavity (65) and comprises at least one recess (118) in the area of its distal end (17'), preferably 4 to 16 recesses (118), the at least one recess (118) being in connection to the pressure unit (2), optionally via the probe channel (13), wherein optionally the at least one recess (118) is provided on an inclined wall portion of the inner side (64) of the housing (16).

3. Device according to claim 2, wherein the housing (16') comprises at least one suction opening at its proximal end, that the probe body (1) comprises at least one further suction channel (128) near its distal end, wherein housing (16') and probe body (1) comprises complementary attachment elements (129) to provide a detachable connection, and wherein the suction opening(s) and the further suction channel(s) (128) are positioned in a way that when the housing (16') is attached at the probe body (1) the suction opening(s) and the further suction channel(s) (128) are providing a medium outlet.

4. Device according to any one of claims 1 to 3, **characterized in that** in an initial position, optionally provided by an abutment or a spring connection (121, 121'), the plug (110, 110') protrudes at least partly from the housing (16, 16') along the distal direction (D).

5. Device according to any one of the preceding claims, **characterized in that** the probe (1) comprises a further probe channel (13') having a distal end (14') which does not extend into the cavity (19) along the distal direction (D) farther than the first named probe channel (13) to avoid being closed in contact with the plug (110, 110'), wherein the further probe channel (13') is connected to a further pressure sensor to determine the pressure in the cavity (19) and to communicate said value to the control unit, wherein the control unit is configured to determine the deformation of the soft tissue (5) as a pressure difference between said pressure sensors of the probe channel (13) and of the further probe channel (13').

6. Device according to any one of the preceding claims, **characterized in that** at least one spring-loaded element (121,121') is arranged in the cavity (19), wherein the spring-loaded element (121,121') connects a proximal end (112, 112') of the plug (110, 110') with a distal end (11) of the probe (1) in such a manner, that the plug (110, 110') is displaceable under the load of the spring in the proximal direction (P).

7. Device according to any one of the preceding claims, **characterized in that** the distal end (111, 111') of the plug (110, 110') being arranged on the side facing the soft tissue (5) has at least at its tip a convex surface (113, 113').

8. Device according to any one of the preceding claims, **characterized in that** the housing (16, 16') is essentially of a cylindrical shape and defines an inner housing diameter (HD), and that the plug (110, 110') is essentially of a cylindrical shape and defines a plug diameter (PD), wherein the inner housing diameter (HD) is larger than the plug diameter (PD), wherein - optionally - a proximal end (112') of the plug (110') has a radial thickening (114) which is arranged in immediate proximity to an inner wall (116, 116') of the housing (16, 16').

9. Device according to any one of the preceding claims, **characterized in that** the proximal end (112) of the plug (110) has an elongation (115), wherein the elongation (115) extends along the proximal direction (P) and is adapted to at least partially surround the probe channel (13) and/or the further probe channel (13').

10. Device according to any one of the preceding claims, further comprising a measuring unit (3, 3', 3", 3"', 3"", 3""', 3""") adapted for measuring a remaining distance (RD) between an initial position, where the plug (110) is first contacting the soft tissue (5), and at least one of the following positions, where the lower closed loop surface (17, 17') of the housing (16) touches the soft tissue (5) and/or where the plug (110) completely covers the distal end (14) of the probe channel (13).

11. Device according to claim 10, **characterized in that** the measuring unit comprises one of the following features:
a) the measuring unit (3) comprises at least one first electrode (31) being in connection with the plug (110) and at least one second electrode (31') being in connection with the probe channel (13), wherein the first electrode (31) and the second electrode (31') increasingly overlap with increasing displacement of the plug (110) along the proximal direction (P) so as to generate an increased capacity;
b) the measuring unit (3') comprises at least one sliding contact (32) being in connection with the plug (110) and a resistance (33) covering the probe channel (13), wherein the resistivity between the sliding contact (32) and the resistance (33) decreases with increasing displacement of the plug (110) along the proximal direction (P);
c) the measuring unit (3") comprises at least one coil (34) being in connection with the plug (110), preferably a plunger coil being in connection with the proximal end (112) of the plug (110), wherein the probe channel (13) comprises a magnetic material, and wherein the inductivity between the coil (34) and the magnetic material of the probe channel (13) increases with increasing displacement of the plug (110) along the proximal direction (P);
d) the measuring unit (3"') comprises a light source (35) and a light detector (36) that are arranged within the cavity (19), wherein the light source (35) sends light to the plug (110) and the light detector (36) detects the light reflected from the plug (110), and wherein the amount of reflected light increases with increasing displacement of the plug (110) along the proximal direction (P);
e) the measuring unit (3"") comprises several membranes (37) that are attached proximally to the plug (110), wherein said membranes (37) are punctured before der plug (110) completely covers the distal end (14) of the probe channel (13), the pressure in the device thereby being neutralized to atmospheric pressure;
f) the measuring unit (3""') comprises a plurality of sealing rings (38), wherein the probe channel (13) comprises a plurality of probe channel perforations (119) and the plug (110) comprises one plug perforation (120), wherein a negative pressure is generated within the probe channel (13) if one of the sealing rings (38) overlies with the plug perforation (120), wherein the negative pressure is neutralized if the plug perforation (120) overlies with the probe channel perforation (119), and wherein the cycle of negative pressure and neutralized pressure is indicative of the displacement of the plug (110) along the proximal direction (P);
or
g) the measuring unit (3""") comprises an additional housing (39) that is arranged displaceably around the housing (16, 16'), wherein the additional housing (39) protrudes over the housing (16, 16') along the distal direction (D), wherein the housing (16, 16') protrudes over the additional housing (39) along the proximal direction (P), and wherein a decreasing protrusion of the housing (16, 16') relative to the additional housing (39) is indicative of the displacement of the additional housing (39) along the proximal direction (P).

12. Device according any one of claims 3 to 11, wherein the control unit (4) is separable from the probe (1), and optionally wherein the pressure unit (2) and / or the pressure sensors are arranged within the control unit (4).

13. Probe housing adapted to be used with a device according to any one of claims 1 to 12, wherein the housing (16') is essentially in the form of a double-walled (63, 64) hollow cylinder having a closed internal cavity (65) and comprises at least one recess (118) in the area of its distal end (17'), preferably 4 to 16 recesses (118), at least one suction opening at its proximal end, preferably at the inner wall, as well as an attachment element (129) to provide a detachable connection at its proximal end, wherein optionally the at least one recess (118) is provided on an inclined wall portion of the inner side (64) of the housing (16).

14. Method for measuring the elastic deformability of soft tissue (5) by applying an under pressure on the soft tissue with a probe (1) according to any one of the preceding claims, the method comprising the steps of:
- optionally, placing the probe (1) in the initial position on the soft tissue (5), wherein only the protruding plug (110, 110') contacts the soft tissue, and wherein the plug (110, 110') starts to displace along the proximal direction (P);
- applying the under pressure once the plug (110, 110') has started to displace along the proximal direction (P);
- displacing the housing (16, 16') along the distal direction (D) until the distal end (17, 17') of the housing contacts the soft tissue, wherein the plug (110, 110') continuously displaces along the proximal direction (P) towards the distal end (14) of the probe channel (13); and
interrupting the under pressure once the plug (110, 110') completely covers the distal end (14) of the probe channel (13) in the final position, wherein the deformability of the soft tissue (5) is measured as a pressure difference between the initial position and the final position.
